Europäisches Patentamt

European Patent Office

Office européen des brevets

(19)

(11) Publication number: **0 298 138**

A1

## EUROPEAN PATENT APPLICATION

(12)

(21) Application number: 87109667.3

(22) Date of filing: 04.07.87

(51) Int. Cl.⁴: **A61N 1/42** , **A61N 5/06**

(43) Date of publication of application:
11.01.89 Bulletin 89/02

(84) Designated Contracting States:
AT BE CH DE ES FR GB GR LI LU NL SE

(71) Applicant: **Castelli, Enzio**
**via Dei Lombardi 6**
**Massa Lombarda Ravenna(IT)**

(72) Inventor: **Castelli, Enzio**
**via Dei Lombardi 6**
**Massa Lombarda Ravenna(IT)**

(74) Representative: **Sassatelli, Franco T., Dr.**
**c/o INIP via Ruggi 5**
**I-40137 Bologna(IT)**

(54) **Pocket device for magnetic therapy.**

(57) The pocket-size instrument consists of a device foreseen for generating an intermittent magnetic flux, with periodicity features at low frequency, capable of producing beneficial effects in organic tissues affected by painful syndrome when the said flux is properly approached and alternatively moved in horizontal sense. At the same time, the above device also generates an intermittent concentric rays light in syntony with the frequency of the magnetic flux, the function of which is to regenerate the epithelial cellules on which the above light is besprinkled. The effect is obtained by means of periodical excitement of a control fed solenoid (B), with electric switch (C), by a low voltage micro-pile (9 Volts) in the circuit of which a LED diode (D) is inserted having the task of a warning lamp and, parallely, an intermittent LED diode (A). By acting on the switch fitted on the stiff case in which the above mentioned elements are contained, the feeding solenoid (B) is closed for generating the magnetic flux which, by means of the intermittent LED, occurs at quick intervals acting on the painful zone to treat along with the red light besprinkling.

FIG.2

## "Pocked device for magnetic therapy".

The present invention consists of a device generating an intermittent magnetic flux, with periodicity feature at low frequency, which can be used in the therapy of organic tissues affected by painful sundrome due different causes. In order to obtain the said effect, the above flux will be properly approached to the relevant anatomical part and roved on it in horizontal sense. Contemporarily with the magnetic flux, the above mentioned device generates an intermittent light with concentric red rays, in syntony with the frequency of the magnetic flux, having the function of regenerating the epithelial cellules on which the same light is besprinkled. This regeneration is obtained by means of periodical excitement of a solenoid which is controlled by means of an electric switch, by a low-voltage battery in the circuit of which a LED diode is inserted having the task of a warning lamp and, parallely, an intermittent diode. By acting on the switch fitted on the stiff case in which the devices are contained, the circuit feeding the solenoid is closed which generates the magnetic flux.

By means of the intermittent LED, this flux takes place at quick intervals on the pianful zone to treat, along with the red light besprinkling.

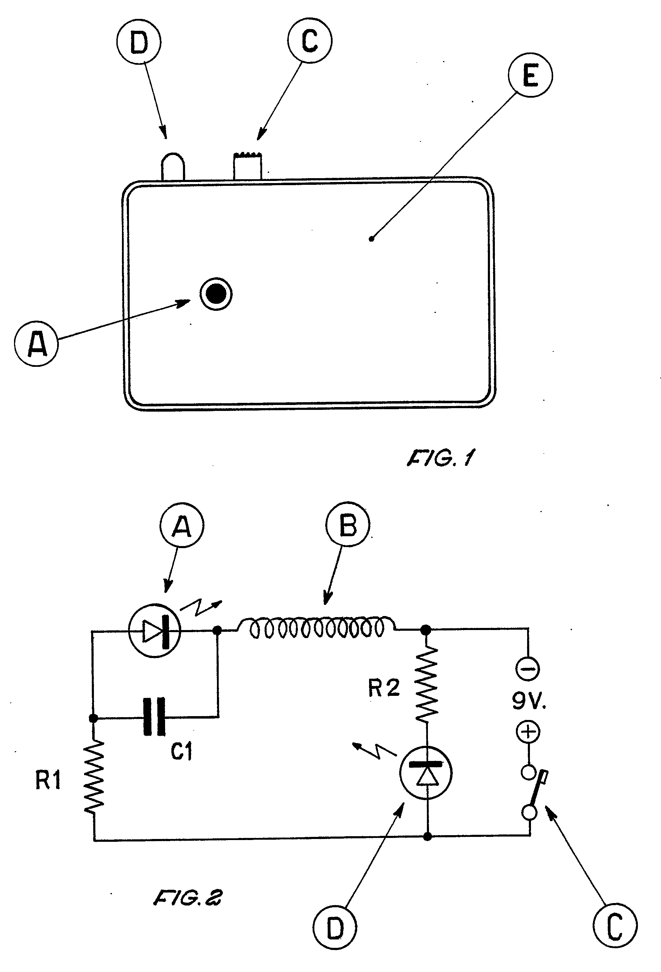

The invention device is illustrated as an example by the drawings of table 1. In fig. 1 the container (E) in insulating material is indicated, the switch (C) , warning light (D) of diode LED and opening (A) for the passage of the red light rays. Fig. 2 is the wiring diagram of the device with micro-pile (9 V.), electric switch (C), solenoid (B), intermittent LED diode (A) connected with the condenser (C1) and resistance (R1).

## Claims

1) Pocked device for magnetic therapy, characterized by the fact that it consists of a solenoid fed by a low tension microbattery (9 V.), foreseen for generating the intermittent magnetic flux for the application. The intermittency of the flux is obtained by inserting a low-frequency intermittent LED diode in the feeding circuit, with the relevant condenser and resistance. The above intermittent LED diode irradiates red light rays on the epidermis to treat.

2) Pocked device for magnetic therapy, as per claim 1, characterized by the fact that another LED diode with the relevant resistance is inserted in the same circuit, the task of which is to light up the warning lamp of the device.

3) Pocked device for magnetic therapy, as per claim 1, characterized by the fact that the device is enclosed in an insulating material container fitted with an electrical switch, in its higher part, for setting to work and stopping the device, whereas in its lower part an opening is foreseen for the passage of the red intermittent light.

FIG.1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-3 045 394 (BANGERT) * Page 9, line 23 - page 14, line 29; figures 1-5 * --- | 1-3 | A 61 N 1/42<br>A 61 N 5/06 |
| A | DE-U-8 532 628 (ELEC SYSTEM VERTRIEBSGESELLSCHAFT FÜR MED. GERÄTE) * Page 4, line 16 - page 5, line 38; figures 1,2 * --- | 1-3 | |
| A | DE-A-3 101 715 (MED-TRONIK) * Page 3, line 25 - page 4, line 4; figures 1,2 * ----- | 1 | |

TECHNICAL FIELDS SEARCHED (Int. Cl.4)

A 61 N

The present search report has been drawn up for all claims

| Place of search | Date of completion of the search | Examiner |
|---|---|---|
| THE HAGUE | 26-01-1988 | SCHMIERER U.J. |